# EUROPEAN PATENT APPLICATION

(11) **EP 3 782 492 A1**
(43) Date of publication of application: **24.02.2021**
(21) Application number: 19193279.7
(22) Date of filing: 23.08.2019
(51) Int. Cl.: A24F 47/00, A61M 11/04, A61M 15/06

(54) **A SUBSTITUTE SMOKING CONSUMABLE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

The present disclosure relates to a heat not burn (HNB) consumable comprising an aerosol-forming substrate housed within a housing. The housing has an inner surface facing the substrate and an opposing outer surface. At least a portion of at least one of the inner and outer surfaces of the housing comprises a moisture resistant surface which may comprise a hydrophobic coating, a textured hydrophobic surface or a layer of dense material.

## Description

### Field of the disclosure

The present disclosure relates to a consumable for a smoking substitute device. In particular, but not exclusively, to a heat not burn consumable. It also relates to a heat not burn system comprising a consumable and a heating element, and a device for housing the system.

### Background

The "smoking" of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful by-products. There have been proposed various smoking substitute systems (or "substitute smoking systems") in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products. Some smoking substitute systems use smoking substitute articles that are designed to resemble a traditional cigarette and are cylindrical in form with a mouthpiece at one end.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories.

There are a number of different categories of smoking substitute systems, each utilising a different smoking substitute approach.

One approach for a smoking substitute system is the so-called "heat not burn" ("HNB") approach in which tobacco (rather than an "e-liquid") is heated or warmed to release vapour. The tobacco may be leaf tobacco or reconstituted tobacco. The vapour may contain nicotine and/or flavourings. In the HNB approach the intention is that the tobacco is heated but not burned, i.e. the tobacco does not undergo combustion.

A typical HNB smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes moisture in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from an inlet to a mouthpiece (outlet), the vapour cools and condenses to form an aerosol for inhalation by the user. The aerosol will normally contain the volatile compounds.

In HNB smoking substitute systems, heating as opposed to burning the tobacco material is believed to cause fewer, or smaller quantities, of the more harmful compounds ordinarily produced during smoking. Consequently, the HNB approach may reduce the odour and/or health risks that can arise through the burning, combustion and pyrolytic degradation of tobacco.

A first existing implementation of the HNB approach is the IQOS™ device from Philip Morris Ltd. The IQOS™ device uses a consumable, including reconstituted tobacco contained within a metallic foil and paper wrapper. The consumable is a cylindrical, rod-shaped consumable designed to resemble a traditional cigarette which is inserted into a heater device. The heater device has a thermally conductive heating blade which penetrates the reconstituted tobacco of the consumable, when the consumable is inserted into the heating device. Activation of the heating device heats the heating element, which, in turn, heats the tobacco in the consumable. The heating of the tobacco causes it to release nicotine vapour and flavours which may be drawn through the mouthpiece by the user through inhalation.

A second existing implementation of the HNB approach is the device known as Glo™ from British American Tobacco. Glo™ also uses a rod-shaped consumable similar in appearance to a traditional cigarette. The consumable includes reconstituted tobacco in a paper wrapping which is heated in a heating device. When the consumable is placed in the heating device, the tobacco is surrounded by a heating element. Activation of the heating device heats the heating element, which, in turn, heats the tobacco in the consumable. The heating of the tobacco causes it to release nicotine vapour and flavours which may be drawn through the consumable by the user through inhalation. The tobacco, when heated by the heating device, is configured to produce vapour when heated rather than when burned (as in a traditional cigarette). The tobacco may contain high levels of aerosol formers (carrier), such as vegetable glycerine ("VG") or propylene glycol ("PG").

Common to both the IQOS™ and Glo™ systems is uneven and incomplete heating of the tobacco, or possible burning of some regions of the tobacco.

Both devices also fail to conceal the residues which remain in the consumable after heating, these residues being both aesthetically unpleasing and also presenting a contamination risk to the user during removal of the consumable from the device.

Furthermore, the aerosol formers may leach from the consumable to stain and/or dampen the paper wrapping which is aesthetically unappealing and which can lead to transfer of the aerosol formers to contaminate the user.

Aspects and embodiments of the disclosure were devised with the foregoing in mind.

### Summary

At its most general, the present disclosure relates to an aerosol-forming article e.g. a smoking substitute article such as an HNB consumable comprising a moisture resistant surface.

In a first aspect, there is provided a heat not burn (HNB) consumable comprising an aerosol-forming substrate housed within a moulded or extruded housing, the housing having an inner surface facing the substrate and an opposing outer surface wherein at least a portion of at least one of the inner and outer surfaces of the housing comprises a moisture resistant surface.

By providing a moisture resistant surface (e.g. a liquid impermeable surface) on the inner and/or outer surface of the housing, leaching of any liquid components of the aerosol-forming substrate through the housing to soil the user is prevented.

Optional features will now be set out. These are applicable singly or in any combination with any aspect.

The aerosol-forming substrate is capable of being heated to release at least one volatile compound that can form an aerosol. The aerosol-forming substrate may be located at the upstream end of the consumable.

As used herein, the terms "upstream" and "downstream" are intended to refer to the flow direction of the vapour/aerosol i.e. with the downstream end of the consumable being the mouth end or outlet where the aerosol exits the consumable for inhalation by the user. The upstream end of the consumable is the opposing end to the downstream end.

In some embodiments, the moisture resistant surface may comprise a hydrophobic coating i.e. at least a portion of at least one of the inner and outer surfaces of the housing may comprise a hydrophobic coating. The hydrophobic coating (especially when provided on the exterior of the housing) is preferably a food grade hydrophobic coating. The hydrophobic coating is preferably a biodegradable hydrophobic coating. The hydrophobic coating may be a wax such as bees wax or carnauba wax.

In other embodiments, the moisture resistant surface may be textured to provide hydrophobic properties e.g. the surface may have a three-dimensional surface structure providing nano- or micrometre scale roughness. The hydrophobic surface protuberances have been found to reduce the effective surface contact area with moisture.

The term "hydrophobic" is used to describe a coating/surface that provides a water contact angle of greater than 70 degrees e.g. greater than 90 degrees at 25°C. In some embodiments, the hydrophobic coating/surface may have a contact angle greater than 120 or 130 or 140 or 150 degrees at 25°C.

In some embodiments, the moisture resistant surface comprises a layer of material (e.g. a layer of biodegradable/plant material such as natural fibre pulp material) having a greater density (lower porosity) than the housing such that the reduced porosity physically limits moisture penetration. For example, the housing and denser moisture resistant layer may comprise the same (plant) material, with the moisture resistant layer having a reduced porosity. The denser moisture resistant layer may have a porosity of less than 20% e.g. less than 10% or less than 5 % or 2% or 1% (where porosity is a measure of void volume to total volume and may be determined using CT scanning or a gas expansion method).

The hydrophobic coating/surface or denser moisture resistant layer may be provided on the inner surface e.g. on the entire inner surface of the housing.

The hydrophobic coating/surface or denser moisture resistant layer may be provided on the outer surface of the housing.

The housing may comprise an outlet/mouthpiece aperture formed at a downstream lateral end of the housing e.g. in a downstream longitudinal end wall of the housing. The hydrophobic coating/surface or denser moisture resistant layer may be provided on the outer surface of the housing surrounding the mouthpiece aperture. It has been found that providing a hydrophobic coating/surface on the exterior of the housing proximal the mouthpiece aperture prevents any adherence of the housing to the user's mouth.

The hydrophobic coating/surface or denser moisture resistant layer may be provided on the entire outer surface of the housing.

The substrate is at least partly (and preferably entirely) enclosed within the housing. The housing may have a non-circular transverse cross-section. The transverse cross-section of the housing may match the transverse cross-section of the substrate (described below).

The housing may be self-supporting. The term "self-supporting" is intended to refer to a housing formed of a material that does not flex or bend under its own weight.

Preferably, the housing is formed of a material that is substantially rigid or semi-rigid i.e. it is not easily flexible.

The paper wrappers provided on the prior art consumables are relatively thin and flimsy. Whilst physically containing the plant product before and after use of the consumable, they do not effectively contain residues in the spent consumable and handling of the spent consumable can result in residue transfer to the user. By providing a more structurally robust (self-supporting) housing, the consumable becomes more akin to a cartridge or "pod" that effectively contains residue after use to protect a user from contamination.

At least a portion and preferably the whole of the housing has a wall thickness in the range of 0.8 to 8.0 mm, e.g. 1.5 to 5.0 mm.

The inner surface of the housing may be textured e.g. it may have a mesh texture.

The housing may be formed at least partly and preferably entirely of a biodegradable material such as cornstarch, bamboo, wood, palm, sugarcane, cardboard or paperboard, recycled or recyclable (thermoplastic) polymer material.

It may be formed of moulded pulp material e.g. natural fibre pulp material. The housing may be at least partly formed of moulded tobacco cellulose pulp, wood pulp, bamboo pump, palm pulp or bagasse pulp. Bagasse pulp is most preferred.

As discussed above, the moisture resistant layer may be formed of the same material as the housing but having a greater density (reduced porosity). Accordingly, the denser moisture resistant layer may be formed of a biodegradable material such as cornstarch, bamboo, wood, palm, sugarcane, cardboard or paperboard, recycled or recyclable (thermoplastic) polymer material.

It may be formed of moulded pulp material e.g. natural fibre pulp material. The denser moisture resistant layer may be at least partly formed of moulded tobacco cellulose pulp, wood pulp, bamboo pump, palm pulp or bagasse pulp. Bagasse pulp is most preferred.

The housing may comprise upper and lower walls spaced by opposing longitudinally-extending transverse walls wherein the depth of the housing (between the upper and lower walls) and the width of the housing (between the opposing transverse walls) are unequal e.g. the width is greater than the depth.

In some embodiments, the upper and lower walls are substantially planar and may be equally spaced by the transverse walls (i.e. the upper and lower walls are parallel to one another) such that the housing is a planar housing.

The opposing transverse walls may be planar and substantially parallel to one another. Where the upper and lower walls are planar, the planar transverse walls may be substantially perpendicular to the upper and lower walls such that the planar housing has a substantially rectangular transverse cross section i.e. the housing is a cuboid housing.

In some embodiments, the housing has at least one curved or rounded wall (e.g. a concave or convex wall) but a non-circular transverse cross section.

For example, at least one and preferably both of the opposing transverse walls may be a curved or rounded wall (e.g. a concave or convex wall).

For example, one or both of the opposing transverse walls may be a substantially convex wall (e.g. a semi-circular wall). Accordingly the planar housing may have a substantially obround transverse cross section i.e. the housing is an obround cylindrical housing.

In some embodiments, one or both of the opposing transverse walls may be a concave wall or may comprise one or more concave portions. For example, the or each curved/rounded opposing transverse wall(s) may each comprise longitudinally-extending upper and lower concave portions which meet at a longitudinally-extending ridge.

The concave portion(s) may be spaced from the planar upper and lower walls by opposing convex portions such that the transverse cross-section is a modified obround where the opposing side edges of the cross-section each take the form of a curly brace/bracket i.e. "{" and "}". Hereinafter, such a housing will be referred to as a "modified obround cylindrical housing".

In other embodiments, the opposing transverse walls may be as described above (i.e. planar, convex, concave or convex and concave) and one or both of the upper/lower walls may be curved/rounded e.g. they may be convex rounded walls. Where the upper and lower walls are convex walls and the transverse walls are convex, the housing may have an oval transverse cross-section. Where the upper and lower walls are convex walls and the transverse walls are planar, the housing may have a truncated oval transverse cross-section. Where the upper and lower walls are convex walls and the transverse walls comprise two concave portions meeting at a longitudinally extending ridge, the housing may have a modified mandorla transverse cross-section.

The chamber within and defined by the inner surfaces of the housing walls may be a cuboid chamber, an obround cylindrical chamber, a modified obround cylindrical chamber or a modified mandorla chamber.

The chamber within the housing preferably has the same transverse cross section as the housing.

Preferably, the transverse cross-section of the housing and the chamber matches the transverse cross-section of the substrate.

The housing may have an at least partly open upstream longitudinal end face. Alternatively, as discussed below, the upstream longitudinal end face may comprise an upstream end wall that at least partly (and preferably fully) obscures the substrate from view.

The opposing downstream longitudinal end wall (which may comprise the outlet/mouthpiece aperture) may comprise a curved/rounded (e.g. a convex/semi-circular) end wall.

At least one (and optionally both) of the opposing transverse walls of the housing may comprise a longitudinally-extending junction such that the housing can be opened to expose the chamber within.

The downstream longitudinal end /wall may also comprise a junction.

For example, both of the opposing transverse walls and the downstream longitudinal end wall could comprise a respective junction such that the housing can be split into two opposing parts allowing for easy insertion during manufacture of the substrate.

Alternatively, one of the opposing transverse walls and the upstream longitudinal end wall may contain the junctions and the other transverse wall may contain a longitudinally extending hinge portion such that the housing may be opened along the junctions by pivoting of the two opposing parts about the hinge portion.

The housing may have a single heating surface (the outer surface of one of the upper and lower walls) for contact with/for facing a heating element (e.g. a planar heating element) or there may be two opposing surfaces (the outer surfaces of both of the upper and lower walls) each for contact with/for facing one of two heating elements (e.g. planar heating elements). The aerosol-forming substrate contained in the housing is then heated externally and inwards through the housing from the heating surface(s) of the upper and/or lower walls.

The substrate may comprise upper and lower surfaces spaced by opposing longitudinally-extending transverse surfaces wherein the depth of the substrate (between the upper and lower surfaces) and the width of the substrate (between the opposing transverse surfaces) are unequal e.g. the width is greater than the depth.

In some embodiments, the upper and lower surfaces are substantially planar and may be equally spaced by the transverse surfaces (i.e. the upper and lower surfaces are parallel to one another) such that the substrate is a planar substrate.

By providing the substrate as a planar substrate rather than as a cylindrical rod (having a substantially circular cross section), the substrate has a greater exposed surface area for contact with a heating element thus allowing quicker and more even heat transfer from the heating element to the plant product. In this manner, heating of the substrate can be effected using a heating element at a lower temperature (e.g. around 250 °C) which reduces the chances of burning of the plant product.

The opposing transverse surfaces may be planar and substantially parallel to one another. Where the upper and lower surfaces are planar, the planar transverse surfaces may be substantially perpendicular to the upper and lower surfaces such that the planar substrate has a substantially rectangular transverse cross section i.e. the substrate is a cuboid substrate.

The transverse cross section is defined by a face having edges defining the width and depth i.e. the term "transverse cross section" is used to denote a cross section through the consumable perpendicular to the longitudinal axis/length of the planar substrate/consumable. The substrate has opposing longitudinal end faces (an upstream end face and a downstream end face) which will each comprise a transverse cross section.

In some embodiments, the substrate has at least one curved or rounded surface but a non-circular transverse cross section.

For example, at least one and preferably both of the opposing transverse surfaces may comprise a curved or rounded surface/surface portion e.g. at least one and preferably both of the opposing transverse surfaces comprises a convex or concave surface/surface portion.

For example, one or both of the opposing transverse surfaces may comprise a substantially convex surface (e.g. a semi-circular surface). Accordingly the planar substrate has a substantially obround transverse cross section i.e. the substrate is an obround cylindrical substrate.

In some embodiments, one or both of the opposing transverse surfaces may be concave or may comprise one or more concave portions. For example, the or each curved/rounded opposing transverse surface(s) may each comprise longitudinally-extending upper and lower concave portions which meet at a longitudinally-extending ridge.

The concave portion(s) may be spaced from the planar upper and lower surfaces by opposing convex portions such that the transverse cross-section is a modified obround where the opposing side edges of the cross-section each take the form of a curly brace/bracket i.e. "{" and "}". Hereinafter, such a substrate will be referred to as a "modified obround cylindrical substrate".

In other embodiments, the opposing transverse surfaces may be as described above (i.e. planar, convex, concave or convex and concave) and one or both of the upper/lower surfaces may be curved/rounded e.g. they may be convex rounded surfaces. Where the upper and lower surfaces are convex surfaces and the transverse surfaces are convex, the substrate may have an oval transverse cross-section. Where the upper and lower surfaces are convex surfaces and the transverse surfaces are planar, the substrate may have a truncated oval transverse cross-section. Where the upper and lower surfaces are convex surfaces and the transverse surfaces comprise two concave portions meeting at a longitudinally extending ridge, the substrate may have a modified mandorla transverse cross-section.

The substrate preferably has a greater width and length than depth. The length and width may be equal but, preferably, the length is greater than the width such that the substrate has substantially rectangular upper and lower surfaces. The length of the substrate (between the upstream and downstream end faces) may be between 10 and 20 mm e.g. between 10 and 15 mm. The width of the substrate (between opposing transverse surfaces) may be between 7 and 18 mm e.g. between 8 and 14 mm or 10 and 12 mm. The depth of the substrate (between the upper and lower surfaces) may be between 1 and 8 mm, e.g. between 2 and 7 mm e.g. around 2 mm or around 6 mm.

In some embodiments, the consumable comprises a single substrate e.g. a single planar substrate as described above. In this case, the depth of the substrate is preferably between 5 and 7 mm e.g. around 6 mm.

The substrate may have a single heating surface (one of the upper and lower surfaces) for contact with/for facing a heating element (e.g. a planar heating element) or there may be two opposing surfaces (both of the upper and lower surfaces) each for contact with/for facing one of two heating elements (e.g. planar heating elements). The substrate is then heated externally and inwards from the upper and/or lower substrate heating surfaces.

In other embodiments, the substrate is heated internally and outwards (towards the upper and lower surfaces).

This may be achieved by providing a penetrable substrate such that a heating element can be inserted into the substrate e.g. into the upstream end face of the substrate.

Alternatively, the substrate may have a hollow core for releasably and slidably receiving the heating element.

In use, the hollow core receives a heating element (i.e. by insertion of the heating element into the hollow core) which can contact the internal surfaces defining the core thus allowing quicker and more even heat transfer from the heating element to the plant product. In this manner, heating of the plant product can be effected using a heating element at a lower temperature (e.g. around 250 °C) which reduces the chances of burning of the plant product.

The hollow core is defined by a longitudinally-extending recess extending from the upstream end face of the substrate. The core recess may extend from the upstream end face to the opposing downstream end face.

The core recess is defined by upper and lower inner surfaces spaced by opposing longitudinally extending inner transverse surfaces. The upper and lower inner surfaces will face the heating element in use.

The depth of the core recess (between the upper and lower inner surfaces) and the width of the recess (between the opposing inner transverse surfaces) are unequal.

In some embodiments, the upper and lower inner surfaces are substantially planar and may be equally spaced by the inner transverse surfaces (i.e. the upper and lower inner surfaces are parallel to one another).

The opposing inner transverse surfaces may be substantially parallel to one another and substantially perpendicular to the upper and lower inner surfaces such that the core recess has a substantially rectangular transverse cross section i.e. the core recess is a cuboid core recess.

In other embodiments, at least one and preferably both of the opposing inner transverse surfaces may comprise a curved or rounded (concave or convex) surface.

For example, one or both of the opposing inner transverse surfaces may comprise a substantially convex surface (e.g. a semi-circular surface) such that the core recess has a substantially obround transverse cross section i.e. the core recess is an obround core recess.

Where the substrate is a hollow cuboid substrate, it may comprise a cuboid core recess. Where the substrate is an obround cylindrical substrate or a modified obround cylindrical substrate, it may comprise an obround core recess.

The recess may have a depth (between the upper and lower inner surfaces) of between 0.5 and 2 mm e.g. around 1 mm. The recess may have a width (between the opposing inner transverse surfaces) of between 7 and 14 mm e.g. between 7 and 12 mm or 8 and 10 mm e.g. around 8 mm. The length of the recess may be between 10 and 20 mm e.g. between 10 and 15 mm.

In these embodiments, the depth of the hollow substrate may be between 4 and 8 mm, e.g. between 5 and 7 mm e.g. around 6 mm.

In other embodiments, the consumable comprises a plurality of substrates e.g. two planar substrates (which may be as described above). Where there are two planar substrates, the depth of each planar substrate is preferably between 1 and 8 mm, e.g. between 2 and 5 mm e.g. around 2 mm.

The planar substrates are preferably aligned and spaced from one another to define a planar recess therebetween such that the consumable has a substantially rectangular transverse cross section.

A heating element can be inserted into the planar recess so as to be releasably housed in the recess. In this way, heat can be transferred quickly and evenly to the plant product via the surfaces defining the planar recess.

In these embodiments, the substrates will each having an inner heating surface facing the planar recess and an opposing outer surface.

The two planar substrates are preferably vertically and horizontally aligned. The planar recess is also vertically and horizontally aligned with the planar substrates.

Where the consumable comprises two planar substrates, each planar substrate may be mounted (e.g. glued) into a respective part (e.g. half) of the housing such that when the two opposing parts are brought together, the planar substrates are spaced from one another to define the planar recess therebetween (as discussed above).

The surfaces defining the core recess or planar recess may be lined with a thermally conductive material. For example, the surface(s) defining the recess may be at least 50% or 60% covered and preferably at least 70 % or 80% or 90% covered. The recess may be fully lined with the thermally conductive material.

The thermally conductive material may be provided as a foil which may be textured e.g. dimpled.

The substrate may comprise at least one channel extending into the plant product from either or both of the upstream and downstream longitudinal end faces of the substrate. The thermally conductive material may extend into the at least one channel. For example, the thermally conductive material may extend from the recess to the at least one channel over the upstream/downstream longitudinal end face of the substrate. This helps increase heat transfer from the heating element within the recess into the substrate.

The consumable may comprise a further layer of the thermally conductive material, or of a further thermally conductive material, on an outer surface of the plant product opposing the recess.

The thermally conductive material or the further thermally conductive material may be selected from the group consisting of: carbon or metal/metal alloy such as aluminium; brass; copper; gold; steel; silver; an alloy of one of more thereof; or a mixture of two or more thereof.

The substrate may be dosed with an e-liquid either in its entirety or in selected portions. For example, the substrate may be dosed with e-liquid at or proximal its heating surfaces.

The substrate may be dosed with e-liquid at its surfaces which face the heating element(s). For example, the substrate may be dosed with e-liquid at or proximal its upper and/or lower surfaces.

Where the substrate is a hollow substrate and comprises a hollow core defined by a core recess, the plant product at or proximal one or more of the upper/lower/transverse inner surfaces defining the core recess may be dosed with e-liquid.

Where the consumable comprises a plurality of planar substrates defining a planar recess, the plant product at or proximal one or both of the surfaces of the planar substrates facing the planar recess may be dosed with e-liquid.

The e-liquid may contain aerosol formers such as polyglycol (PG) and/or vegetable glycerine (VG). It may contain flavourings.

The consumable may further comprise a filter downstream element downstream of the substrate. The downstream element may have a non-circular transverse cross-section. The transverse cross-section of the downstream element may match the transverse cross-section of the substrate.

The downstream element may be a porous element. The porous element may have a porosity such that it at least partly blocks the passage (filters out) at least one of the components of the aerosol/vapour. Thus the downstream porous element may be a filter element. In other embodiments, the porous element may have a density/porosity/permeability such that it is permeable to (allows the passage of) all components of the aerosol/vapour.

In yet further embodiments, the downstream element may be a solid element having a hollow bore for the passage of the aerosol/vapour.

The downstream element (e.g. the filter element) may comprise upper and lower surfaces spaced by opposing longitudinally-extending transverse surfaces wherein the depth of the downstream/filter element (between the upper and lower surfaces) and the width of the downstream/filter element (between the opposing transverse surfaces) are unequal.

In some embodiments, the upper and lower surfaces are substantially planar and may be equally spaced by the transverse surfaces (i.e. the upper and lower surfaces are parallel to one another) such that the downstream/filter element is a planar downstream/filter element.

The opposing transverse surfaces may be substantially parallel to one another and substantially perpendicular to the upper and lower surfaces such that the planar downstream/filter element has a substantially rectangular transverse cross section i.e. the downstream/filter element is a cuboid downstream/filter element.

In other embodiments, at least one and preferably both of the opposing transverse surfaces may comprise a curved or rounded (concave or convex) surface.

For example, one or both of the opposing transverse surfaces may comprise a substantially convex surface (e.g. a semi-circular surface) such that the downstream/filter element has a substantially obround transverse cross section i.e. the downstream/filter element is an obround cylindrical downstream/filter element.

In some embodiments, one or both of the opposing transverse surfaces may be concave or may comprise one or more concave portions. For example, the or each curved/rounded opposing transverse surface(s) may each comprise longitudinally-extending upper and lower concave portions which meet at a longitudinally-extending ridge.

The concave portion(s) may be spaced from the planar upper and lower surfaces by opposing convex portions such that the transverse cross-section is a modified obround where the opposing side edges of the cross-section each take the form of a curly brace/bracket i.e. "{" and "}". Hereinafter, such a downstream/filter element will be referred to as a "modified obround cylindrical downstream/filter element".

In other embodiments, the opposing transverse surfaces may be as described above (i.e. planar, convex, concave or convex and concave) and one or both of the upper/lower surfaces may be curved/rounded e.g. they may be convex rounded surfaces. Where the upper and lower surfaces are convex surfaces and the transverse surfaces are planar, the downstream/filter element may have a truncated oval transverse cross-section. Where the upper and lower surfaces are convex surfaces and the transverse surfaces are convex, the downstream/filter element may have an oval transverse cross-section. Where the upper and lower surfaces are convex surfaces and the transverse surfaces comprise two concave portions meeting at a longitudinally extending ridge, the downstream/filter element may have a modified mandorla transverse cross-section.

The downstream/filter element preferably has a greater width and length than depth. The depth of the downstream/filter element may be between 4 and 8 mm, e.g. between 5 and 7 mm e.g. around 6 mm. The width of the downstream/filter element may be between 7 and 18 mm e.g. between 8 and 14 mm or 10 and 12 mm. The length of the downstream/filter element may be between 2mm and 25 mm e.g. between 3mm and 22mm.

The downstream/filter element has an upstream longitudinal end face which faces and may abut the downstream longitudinal end face of the substrate. The inner surface of the downstream longitudinal end wall of the housing may abut the downstream longitudinal end surface of the downstream/filter element.

The downstream longitudinal end face of the downstream/filter element may comprises a curved/rounded surface (e.g. a convex surface such as a semi-circular surface).

The downstream/filter element may comprise a hollow bore. The hollow bore may extend from the upstream longitudinal end face of the downstream/filter element to the downstream longitudinal face of the downstream/filter element.

The hollow bore may have a circular, rectangular or obround transverse cross sectional area. The bore may have a uniform transverse cross-sectional area.

The downstream/filter element may be comprised of cellulose acetate or polypropylene tow. The downstream/filter element may be comprised of activated charcoal. The downstream/filter element may be comprised of paper. The downstream/filter element may be comprised of plant material e.g. extruded or pressed plant material e.g. pressed/extruded pulp material e.g. bagasse. The downstream/filter element may be circumscribed with a plug wrap e.g. a paper plug wrap.

In some embodiments, the downstream/filter element may comprise at least one liquid release member.

The liquid release member can comprise an envelope for containing the liquid. The envelope can be rigid and fragmentable under pressure (e.g. upon contact with the heating element). Alternatively, the envelope can be meltable upon application of heat. Alternatively, the envelope can be dissolvable in the aerosol.

The liquid release member may contain an aerosol former such as vegetable glycerine and/or propylene glycol. By containing the aerosol former within a liquid release member that is configured to release the liquid (e.g. aerosol former) upon use, seepage of the liquid from the consumable to contaminate the user is avoided. The liquid release member may comprise a flavouring.

The liquid release member may positioned proximal the abutment between the downstream/filter element (e.g. at the upstream longitudinal end face of the downstream/filter element) and the substrate (i.e. the downstream longitudinal end face of the downstream/filter element) so that upon release, the liquid can penetrate the plant product in the substrate.

The consumable may comprise a spacer e.g. a paper/cardboard spacer interposed between the downstream/filter element and the substrate. The spacer defines a space or cavity or chamber downstream from the aerosol-forming substrate. For example, it may be provided between the aerosol-forming substrate and the downstream/filter element. The spacer acts to allow both cooling and mixing of the aerosol.

The spacer may be a planar spacer e.g. having a substantially rectangular or substantially obround transverse cross section. The spacer may have a transverse cross-section such as any of the transverse cross-sections defined for the substrate/housing/downstream element. The spacer may have a transverse cross-section substantially matching the transverse cross section of the substrate and/or downstream/filter element and/or housing.

The spacer preferably has a greater width and length than depth. The length and width may be equal but, preferably, the width is greater than the length. The depth of the spacer may be between 4 and 8 mm, e.g. between 5 and 7 mm e.g. around 6 mm. The width of the spacer may be between 7 and 18 mm e.g. between 8 and 14 mm or 10 and 12 mm.

The consumable may further comprise a wrapping e.g. a paper or cardboard wrapping that encloses the upper and lower surfaces and the transverse walls of the substrate (and downstream/filter element and/or spacer where present).

In embodiments where the substrate comprises at least one channel extending into the plant product from the upstream longitudinal end face of the substrate (as described above), the wrapping e.g. the cardboard wrapping may comprise a transverse extension which extends to cover a portion of the upstream longitudinal end face of the substrate. The transverse extension may then comprise an inwardly-depending axial extension extending inwards into the at least one channel in the substrate.

As discussed above, the downstream longitudinal end of the housing comprises a downstream longitudinal end wall. The downstream/filter element is typically provided adjacent e.g. with its downstream longitudinal end face abutting this longitudinal end wall of the housing. Thus the downstream longitudinal end wall at least partly (and preferably completely) obscures/conceals the downstream/filter element from view by the user.

By concealing the downstream/filter element from view, the user is not exposed to the residues remaining in the consumable after use thus improving the aesthetic appeal of the consumable after use and avoiding transfer of residue to the user.

Although the downstream longitudinal end wall may comprise one or more outlet(s)/mouthpiece aperture(s), this/these are typically small enough that visual inspection of the downstream/filter element is significantly impeded compared to the prior art consumable where the end face of the downstream/filter element is completely exposed. Thus whilst the downstream longitudinal wall may be discontinuous, it preferably covers (e.g. overlies or abuts) at least 20% e.g. at least 30 or 40 % and preferably at least 50%, e.g. at least 70% such as at least 80% or 90% of the surface area of the downstream longitudinal end face of the downstream/filter element.

Similarly, the upstream longitudinal end face may comprise an upstream longitudinal end wall that at least partly (e.g. fully) obscures the substrate from view at least prior to use.

The upstream longitudinal end face of the housing may comprise an upstream longitudinal end wall for at least partly overlying (e.g. abutting) the upstream longitudinal end face of the substrate. The upstream longitudinal end wall may comprise an aperture (into which the heating element can be inserted).

The upstream longitudinal end wall may be a perimeter wall i.e. it may extend only around one or more of the edges of the upstream longitudinal end face of the housing. For example, it may extend around all edges to form a frame defining the aperture (into which the heating element can be inserted). The aperture may be dimensioned to match the dimensions of the hollow core recess when the substrate is a hollow core substrate.

In other embodiments, the upper longitudinal end wall of the housing may extend along the upper and lower edges to form rails defining the aperture therebetween. The aperture may be dimensioned to match the dimensions of the planar recess when the consumable comprises two planar substrates.

In embodiments where the substrate comprises at least one channel extending into the plant product from the upstream longitudinal end face of the plant product (as described above), the upstream longitudinal end wall may comprise an inwardly-depending axial extension, extending inwards into the at least one channel in the substrate.

The upstream longitudinal end face of the housing may additionally or alternatively comprise a pierceable or peelable membrane such as a metallic foil or plastic membrane. The membrane may be mounted across the entire open upstream longitudinal end face of the housing or it may be mounted on the upstream longitudinal end wall across the aperture. The membrane seals the upstream longitudinal end face prior to use and is pierced to mount the consumable on the heating element.

In order to generate an aerosol, the substrate comprises at least one volatile compound that is intended to be vaporised/aerosolised and that may provide the user with a recreational and/or medicinal effect when inhaled. Suitable chemical and/or physiologically active volatile compounds include the group consisting of: nicotine, cocaine, caffeine, opiates and opoids, cathine and cathinone, kavalactones, mysticin, beta-carboline alkaloids, salvinorin A together with any combinations, functional equivalents to, and/or synthetic alternatives of the foregoing.

The plant material may comprise least one plant material selected from the list including *Amaranthus dubius*, *Arctostaphylos uva-ursi* (Bearberry), *Argemone mexicana*, *Amica*, *Artemisia vulgaris*, Yellow Tees, *Galea zacatechichi*, *Canavalia maritima* (Baybean), *Cecropia mexicana* (Guamura), *Cestrum noctumum*, *Cynoglossum virginianum* (wild comfrey), *Cytisus scoparius*, *Damiana*, *Entada rheedii, Eschscholzia califomica* (California Poppy), *Fittonia albivenis*, *Hippobroma longiflora*, *Humulus japonica* (Japanese Hops), *Humulus lupulus* (Hops), *Lactuca virosa* (Lettuce Opium), *Laggera alata*, *Leonotis leonurus*, *Leonurus cardiaca* (Motherwort), *Leonurus sibiricus* (Honeyweed), *Lobelia cardinalis*, *Lobelia inflata* (Indian-tobacco), *Lobelia siphilitica*, *Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum*, *Salvia dorrii* (Tobacco Sage), Salvia species (Sage), *Scutellaria galericulata*, *Scutellaria lateriflora*, *Scutellaria nana*, *Scutellaria* species (Skullcap), *Sida acuta* (Wireweed), *Sida rhombifolia*, *Silene capensis*, *Syzygium aromaticum* (Clove), *Tagetes lucida* (Mexican Tarragon), *Tarchonanthus camphoratus*, *Tumera diffusa* (Damiana), *Verbascum* (Mullein), *Zamia latifolia* (Maconha Brava) together with any combinations, functional equivalents to, and/or synthetic alternatives of the foregoing.

Preferably, the plant material is tobacco. Any type of tobacco may be used. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

Any suitable parts of the tobacco plant may be used. This includes leaves, stems, roots, bark, seeds and flowers.

The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon).

The aerosol-forming substrate may comprise reconstituted tobacco. The substrate, especially the hollow core substrate may be formed by extrusion.

Extruded tobacco can produced by forming a liquid mixture of powered tobacco and a binding agent such as a gum (e.g. xanthan, guar, arabic and/or locust bean gum). The liquid mixture is heated and then extruded through a die. The extrudate is then dried. Flavouring may be added to the liquid mixture prior to extrusion to provide a flavoured extruded substrate e.g. a flavoured extruded hollow core substrate.

The flavourant may be provided in solid or liquid form. It may include menthol, liquorice, chocolate, fruit flavour (including e.g. citrus, cherry etc.), vanilla, spice (e.g. ginger, cinnamon) and tobacco flavour.

The aerosol-forming substrate may comprise one or more additives selected from humectants, flavourants, fillers, aqueous/non-aqueous solvents and binders.

Humectants are provided as vapour generators - the resulting vapour helps carry the volatile active compounds and increases visible vapour. Suitable humectants include polyhydric alcohols (e.g. propylene glycol (PG), triethylene glycol, 1,2-butane diol and vegetable glycerine (VG)) and their esters (e.g. glycerol mono-, di- or tri-acetate). They may be present in the aerosol-forming substrate in an amount between 1 and 50 wt%.

The humectant content of the aerosol-forming substrate may have a lower limit of at least 1 % by weight of the plant material, such as at least 2 wt %, such as at least 5 wt %, such as at least 10 wt %, such as at least 20 wt %, such as at least 30 wt %, or such as least 40 wt %.

The humectant content of the aerosol-forming substrate may have an upper limit of at most 50 % by weight of the plant material, such as at most 40 wt %, such as at most 30 wt %, or such as at most 20 wt %.

Preferably, the humectant content is 1 to 40 wt % of the aerosol-forming substrate, such as 1 to 20 wt %

Suitable binders are known in the art and may act to bind together the components forming the aerosol-forming substrate. Binders may comprise starches and/or cellulosic binders such as methyl cellulose, ethyl cellulose, hydroxypropyl cellulose, hydroxyethyl cellulose and methyl cellulose, gums such as xanthan, guar, arabic and/or locust bean gum, organic acids and their salts such as alginic acid/ sodium alginate, agar and pectins.

Preferably the binder content is 5 to 10 wt% of the aerosol-forming substrate e.g. around 6 to 8 wt%.

Suitable fillers are known in the art and may act to strengthen the aerosol-forming substrate. Fillers may comprise fibrous (non-tobacco) fillers such as cellulose fibres, lignocellulose fibres (e.g. wood fibres), jute fibres and combinations thereof.

Preferably, the filler content is 5 to 10 wt% of the aerosol-forming substrate e.g. around 6 to 9 wt%.

The aerosol-forming substrate may comprise an aqueous and/or non-aqueous solvent. In some embodiments, the aerosol forming substrate has a water content of between 5 and 10 wt% e.g. between 6-9 wt% such as between 7-9 wt%.

The flavourant may be provided in solid or liquid form. It may include menthol, liquorice, chocolate, fruit flavour (including e.g. citrus, cherry etc.), vanilla, spice (e.g. ginger, cinnamon) and tobacco flavour. The flavourant may be evenly dispersed throughout the aerosol-forming substrate or may be provided in isolated locations and/or varying concentrations throughout the aerosol-forming substrate.

The consumable described above may be coupled with a heating element in a heat not burn (HNB) device.

Accordingly in a second aspect, there is provided a heat not burn (HNB) system comprising:
a heat not burn consumable as described above in the first aspect; and
a device comprising at least one heating element.

The device may be a HNB device i.e. a device adapted to heat but not combust the aerosol-forming substrate.

The device may comprise a device housing for housing the heating element(s). The heating element(s) may comprise an elongated e.g. rod, tube-shaped or blade heating element. The heating element(s) may project into or surround a cavity within the device housing for receiving the consumable described above.

The device may further comprise a PCB connected to the heating element(s) for controlling the temperature of the heating element(s). It may further comprise a battery e.g. a recyclable battery such as a 2000mAh battery.

In some embodiments, the device comprises a first heating element for facing/abutting/overlying the upper or lower surface of the substrate or housing. The device may comprise a second heating element which, when the consumable is engaged, faces/abuts/overlies the other of the upper and lower surface of the substrate or housing.

In some embodiments, the device comprises a core heating element for penetrating the substrate or for being received in the hollow core recess of the substrate.

The at least one heating element (e.g. first/second/core heating element) may be a planar heating element. It may have a greater width and length than depth. The length and width may be equal but, preferably, the length is greater than the width such that the planar heating element is a rectangular element i.e. has a substantially rectangular upper and lower planar surfaces. The length of the planar heating element may be between 10 and 20 mm e.g. between 10 and 15 mm. The width of the planar heating element may be between 7 and 14 mm e.g. between 7 and 12 mm or 7 and 10 mm e.g. around 8 mm. The depth of the planar heating element may be between 0.5 and 2 mm, e.g. around 1 mm.

The first/second/core heating element may be a ceramic heating element.

The heat not burn (HNB) device may comprise:
a device housing; and
at least one heating element, the at least one heating element being housed within a cavity at a first longitudinal end of the device housing, the device housing have a first longitudinal end face defining an aperture in communication with said cavity,
wherein the device further comprises a sealing plate movable from a first position in which the aperture is open to a second position in which the aperture is at least partially sealed by the sealing plate.

The sealing plate may be slidable (e.g. slidable in an axial direction) from the first position to the second position.

In the first position, the sealing plate forms a base of the cavity with the at least one heating element extending towards the aperture through the sealing plate. The sealing plate may be an apertured plate, so that as the sealing plate moves from the first to the second position, the at least one heating element passes through the aperture.

The device housing may comprise at least one channel and the sealing plate may comprise at least one transverse tab extending from the sealing plate through the channel to rest on an exterior of the device housing. The device housing may comprise two opposing channels and the sealing plate may comprise two opposing transverse tabs. The transverse tab(s) may be used to manually move the sealing plate between the first and second positions.

The device housing (and the cavity) may have a substantially rectangular or obround transverse cross-section.

The device is adapted to receive a consumable (which is as described above) and which is insertable into the device housing for engagement with the at least one heating element (which may be a first/second/core heating element as described above). Where the consumable comprises a housing, the consumable is inserted with the second longitudinal end wall of the housing protruding from the device housing.

The consumable is inserted when the sealing plate is in its first position. After use, the sealing plate is moved to its second position which forces the consumable from the chamber and, ultimately blocks the aperture at the first longitudinal end face of the device housing so that the user is prevent from contacting the hot heating element.

In a third aspect, there is provided a method of using a heat not burn system according to the second aspect, the method comprising:
inserting the consumable into the device; and
heating the consumable using the heating element.

In some embodiments, the method comprises inserting the consumable into a cavity within the device housing and penetrating the consumable with a core heating element upon insertion of the consumable. For example, the core heating element (e.g. the planar core heating element) may penetrate the aerosol-forming substrate in the consumable e.g. by being received within the hollow core recess/planar recess of the substrate.

The core heating element may be received in the housing through the upstream longitudinal end face of the housing. Where there is an upstream longitudinal end wall, the core heating element may be received in the housing through the aperture. Where there is a membrane/foil sealing the upstream longitudinal end face of the housing or the aperture, the membrane is removed or pierced to allow insertion of the core heating element into the housing.

In other embodiments, the method comprises inserting the consumable into the cavity within the device housing so that the first heating element overlies the upper surface of the substrate e.g. in abutment with the upper wall of the housing. The method may further comprise inserting the consumable into the cavity so that the second heating element overlies the lower surface of the substrate e.g. in abutment with the lower wall of the housing.

Once consumed the consumable may be released from the or each heating element and a further consumable may subsequently be (releasably) engaged with the or each heating element of the device for heating.

The disclosure includes the combination of the aspects and preferred features described except where such a combination is clearly impermissible or expressly avoided.

### Summary of the figures

So that the disclosure may be more readily understood, and so that further features thereof may be appreciated, embodiments and experiments illustrating the principles of the disclosure will now be described by way of example with reference to the accompanying figures in which:
Figure 1 shows cuboid brick of plant product;
Figure 2 shows a housing for a consumable;
Figure 3 shows a second embodiment of a consumable comprising a planar slab of plant product;
Figures 4a - 4c show a third embodiment of a consumable;
Figures 5a and 5b show a fourth embodiment of a consumable;
Figure 6 shows the fourth embodiment with a core heating element inserted;
Figure 7 shows a lateral cross section through the third embodiment with a core heating element inserted;
Figures 8 - 10 show a device according to an embodiment;
Figure 11 shows a fifth embodiment of a consumable;
Figure 12 shows a longitudinal cross section through a sixth embodiment of a consumable;
Figure 13 shows a longitudinal cross section through a seventh embodiment of a consumable;
Figure 14 shows a perspective internal view of an eighth embodiment of a consumable; and
Figure 15 shows the downstream longitudinal end wall of the housing of the embodiment shown in

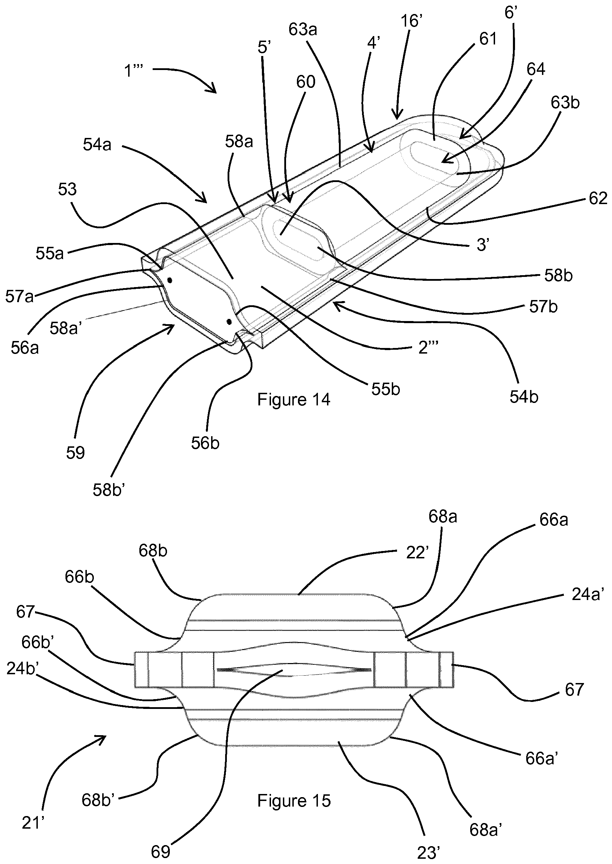

Figure 14; and

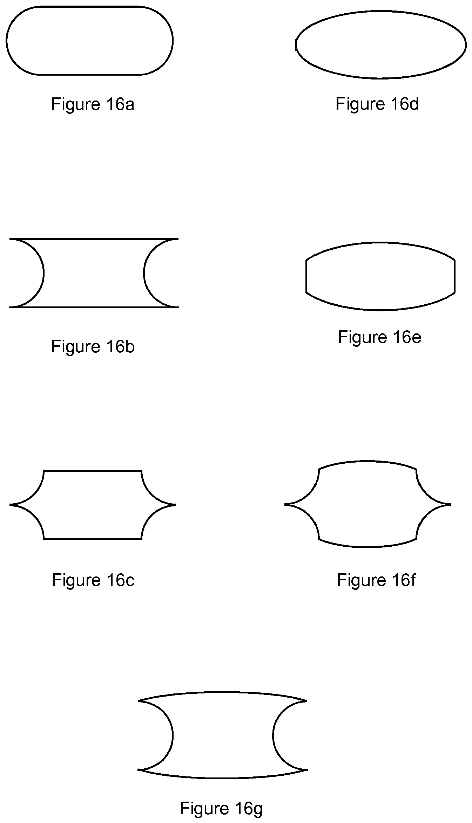

Figures 16a - 16g show alternative transverse cross sections suitable for the aerosol-forming substrate, downstream/filter element or housing.

### Detailed Description

Aspects and embodiments of the disclosure will now be discussed with reference to the accompanying figures. Further aspects and embodiments will be apparent to those skilled in the art.

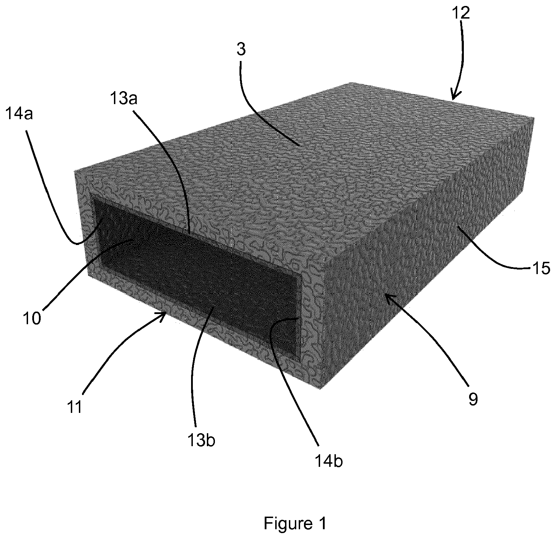

Figure 1 shows a portion of reconstituted tobacco extruded into a cuboid brick 9. The cuboid brick has an upper surface 3 and a lower surface (not visible) spaced by opposing transverse surfaces 15 (only one visible).

The length of the cuboid brick is typically around 12 mm, with a depth of around 6 mm and width of around 10 mm.

The cuboid brick 9 has a hollow core defined by a cuboid recess 10 extending in a length direction from the upstream longitudinal end face 11 of the cuboid brick 9 to the opposing downstream longitudinal end face 12.

The cuboid recess 10 is defined by upper and lower inner surfaces 13a, 13b and opposing inner transverse surfaces 14a, 14b. The cuboid recess has a depth of around 1 mm, a width of around 8 mm and a length of around 12 mm.

The reconstituted tobacco at or proximal one or more of the upper/lower/transverse inner surfaces 13a, 13b, 14a, 14b defining the cuboid recess 10 may be dosed with an e-liquid which may contain aerosol formers such as polyglycol (PG) and/or vegetable glycerine (VG).

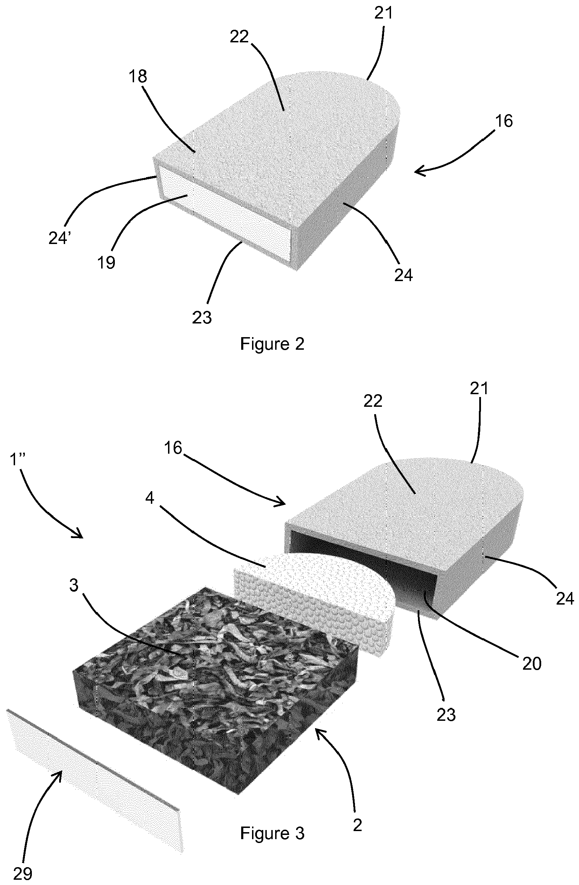

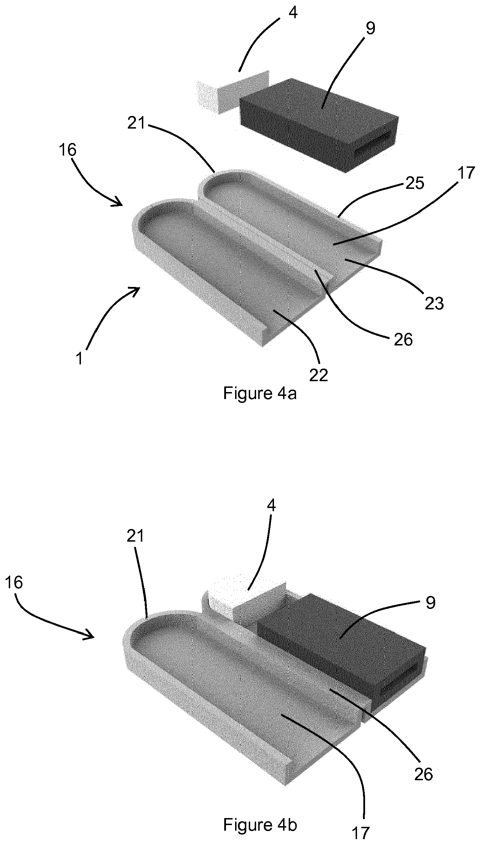

As can be seen in Figures 4a-4c, the cuboid brick 9 can be inserted and glued into a substantially rigid, self-supporting housing 16 having walls of a uniform thickness of around 2 mm formed of moulded bagasse pulp to form the consumable 1. The housing 16 has a meshed inner surface 17 facing the reconstituted tobacco. The outer surface 18 (visible in Figure 2) of the housing 16 is substantially smooth.

As also seen in Figure 2, the housing 16 is a hollow cuboid housing defining a cuboid chamber 20 which is dimensioned to receive the cuboid brick 9 of reconstituted tobacco.

The housing 16 has an open upstream longitudinal end face 19 having a rectangular transverse cross-section. It has a rounded opposing downstream longitudinal end wall 21 which has at least one mouthpiece aperture (not visible).

The consumable 1 has a filter 4 having a rounded downstream longitudinal end face for abutment with the inside surface of the downstream longitudinal end wall 21 of the housing 16.

The downstream longitudinal end wall 21 at least partly (and preferably completely) obscures the filter 4 from view by a user.

By concealing the filter 4 from view, the user is not exposed to the residues remaining in the filter 4/consumable 1 after use thus improving the aesthetic appeal of the consumable after use and avoiding transfer of residue to the user.

The housing 16 comprises an upper wall 22 and lower wall 23 spaced by opposing transverse walls 24. One of the opposing transverse walls 24 comprises a longitudinally extending junction 25 and the downstream longitudinal end wall 21 also comprises a junction such that the housing can be opened to expose the cuboid chamber as shown in figures 4a-4c.

The other opposing transverse wall 24' comprises a longitudinally extending hinge portion 26 such that the housing 16 may be opened along the junctions by pivoting of the upper wall 22 and lower wall 23 about the hinge portion 26.

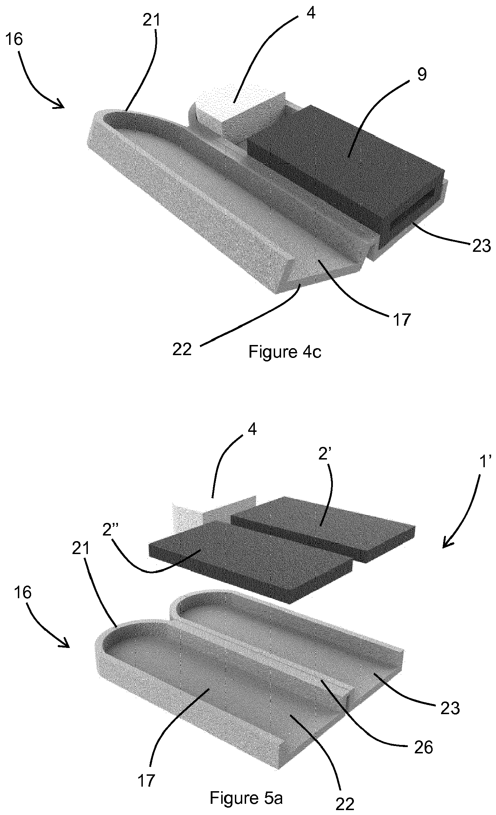

In this way, the cuboid brick 9 and the filter 4 can be fitted (and glued) into the opened housing 16 and then the housing closed (by pivoting about the hinge portion 26 as shown in Figure 4c). The junctions can be sealed e.g. with glue.

The closed housing 16 has the shape shown in Figure 2 and, with such a housing 16, the consumable 1 becomes more akin to a cartridge or "pod" that effectively contains residue after use to protect a user from contamination. Furthermore, the housing 16 is provided on its outer surface i.e. on the outer surface of the upper wall 22, lower wall 23, opposing transverse walls 24 and downstream longitudinal end wall 21 with a hydrophobic surface or coating. This helps prevent transfer of the e-liquid from the substrate (i.e. cuboid brick 9) to the user. It also helps prevent tackiness around the mouthpiece aperture in the downstream longitudinal end wall 21.

Figures 5a-5b show another embodiment in which the consumable 1' comprises two planar substrates 2', 2" of reconstituted tobacco, each having a depth of around 2 mm, a length of around 12 mm and a width of around 10 mm.

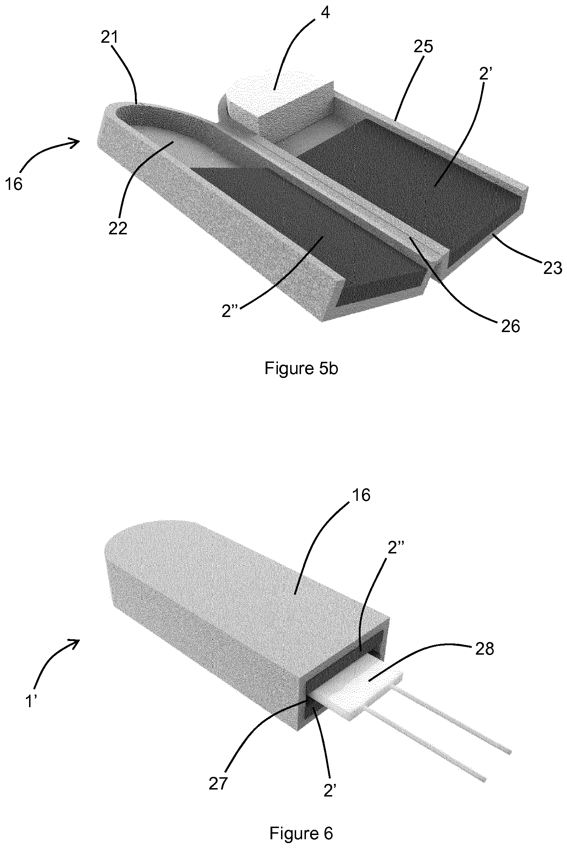

The two planar substrates 2', 2" are vertically and horizontally aligned and spaced from one another to define a planar recess 27 (visible in Figure 6). The housing 16 is as described previously and the two planar substrates 2', 2" are each glued into a respective half of the housing 16 (see Figure 5b) before closing the housing 16 by pivoting along the hinge portion 26 to form the planar recess 27. The tobacco at or proximal the inner surface (facing the planar recess 27) on one or both of the planar slabs 2', 2" is dosed with e-liquid as described above. The inner surface of the housing 16 is coated with a hydrophobic coating or textured to provide a hydrophobic surface to prevent seepage of the e-liquid from the substrates 2', 2" through the housing. The junctions around the housing 16 are sealed using glue.

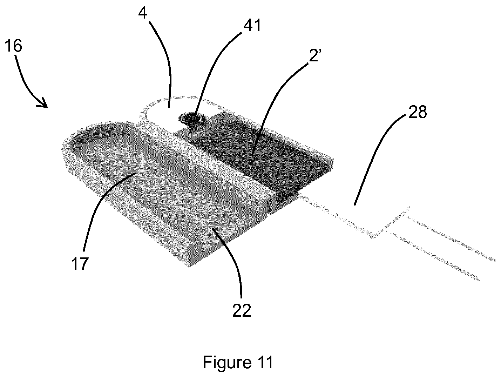

Figure 11 shows a variation (one planar substrate omitted from view) where the filter 4 (shown in cross-section) comprises a liquid release member 41 containing an aerosol former such as vegetable glycerine and/or propylene glycol at its first longitudinal end face. The release member 41 is configured to release the aerosol former during use (e.g. by providing an envelope that is breakable upon abutment with a heating element 28 or that melts during heating) so that upon release, the aerosol former can penetrate the plant product.

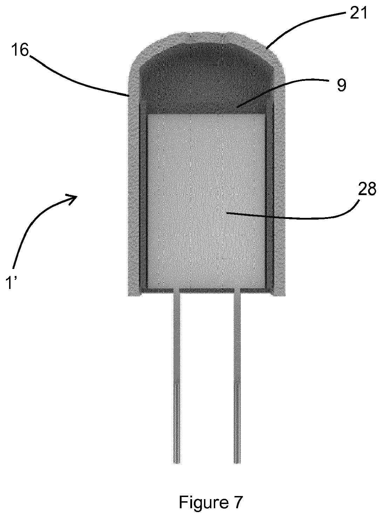

The closed housing 16 is seen in Figure 6 with a planar, ceramic heating element 28 inserted into the planar recess 27. Figure 7 shows a longitudinal cross section of the housing 16 containing the cuboid brick 9 of reconstituted tobacco.

As can be seen, when inserted into the recess 27, the heating element 28 can contact the surfaces of the cuboid recess 10 or planar recess 27 thus allowing quicker heating. It can also be seen that, in all embodiments, the depth of the reconstituted tobacco between the heating surface(s) and the opposing surfaces is substantially constant in the depth direction which results in a more even heat transfer from the heating element 28 to the reconstituted tobacco. In this manner, heating of the tobacco can be effected using a heating element 28 at a lower temperature (e.g. around 250 °C) which reduces the chances of burning of the plant product.

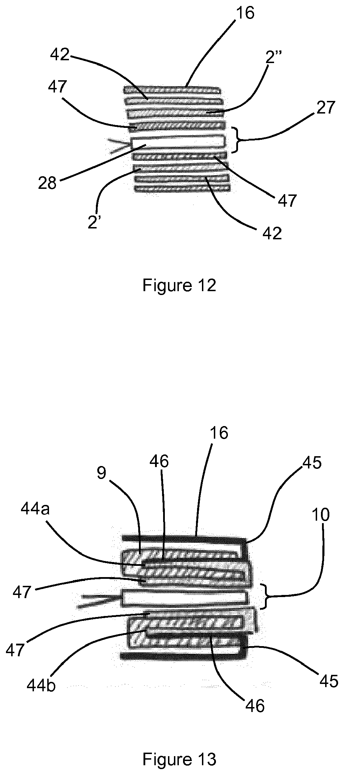

Figures 12 and 13 show further embodiments where the planar recess 27 or cuboid recess 10 is fully lined with a thermally conductive material such as aluminium foil 47. The aluminium foil 47 fully lines the recess 10, 27 and overlies the plant product which may be in the form of two planar substrates 2', 2" (as shown in Figure 12) or may be a cuboid brick 9 (as shown in Figure 13).

In the embodiment shown in Figure 12, the opposing outer surfaces of the planar substrates 2', 2" are further lined with a dimpled foil 42 (which is liquid impermeable). The plant product and foil layers 47, 42 are enclosed with moulded bagasse pulp housing 16.

The heating element 28 is received within the planar recess 27 and the aluminium foil 47 increases heat transfer to the plant product. The dimpled foil 42 forms a liquid impermeable barrier to prevent seepage of any e-liquid dosed into the plant product into the cardboard wrapper, the dimples acting to increase air flow through the consumable upon inhalation by the user.

In Figure 13, the aluminium foil 47 fully lines the cuboid recess 10 and then extends over the downstream longitudinal end face 12 of the cuboid brick 9 and axially into channels 44a, 44b formed within the downstream longitudinal end face 12. Thus the aluminium foil 47 partly covers the downstream longitudinal end face 12 of the cuboid brick.

The cuboid brick 9 and foil layers 47, 42 are enclosed with the moulded bagasse pulp housing 16. The housing 16 comprises a transverse extension 45 which extends to cover a portion of the downstream longitudinal end face 12 of the cuboid brick 9. The transverse extension 45 comprises an inwardly-depending axial extension 46 extending inwards into the channels 44a, 44b in the plant product in abutment with the aluminium foil 47.

The heating element 28 is received within the cuboid recess 10 and the aluminium foil 47 increases heat transfer to the plant product.

A further embodiment of a consumable 1" is shown in Figure 3 where the housing 16 and filter 4 are as previously described. The reconstituted tobacco is formed as single planar substrate 2 having a substantially rectangular upper surface 3. The substrate 2 has a length of around 12 mm, a width of around 8 mm and a depth of around 6 mm.

The upstream longitudinal end face 19 of the housing 16 is provided with a pierceable or peelable membrane such as a metallic foil or plastic membrane 29. Such a membrane may be provided on any of the previously described embodiments and is provided to at least partly obscure the reconstituted tobacco from view by a user prior to use. When the consumable 1"' is used, the membrane can be removed or pierced to insert the heating element 28.

For this embodiment, the heating element could be a heating blade that pierces the planar substrate 2.

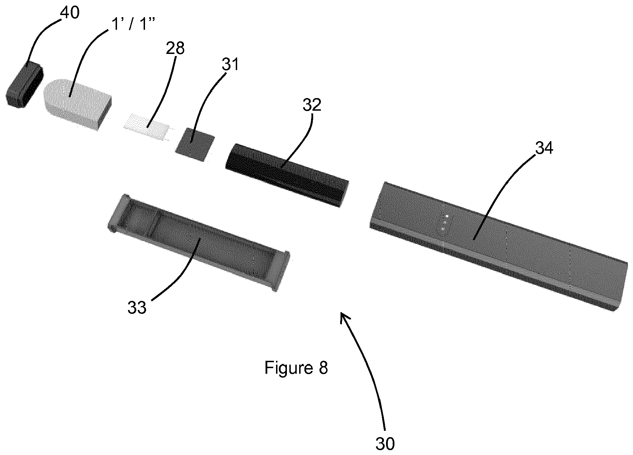

Figure 8 shows a heat not burn (HNB) device 30 comprising the heating element 28 which is mounted on and controlled by a PCB 31 connected to a battery 32, the PCB 31 and battery 32 being housed within an electrical sleeve 33. In turn electrical sleeve 33 and heating element 28 are housed within (and fully enclosed by) a device housing 34. The device housing 34 has a chamber 35 at its first longitudinal end which has an aperture at its first longitudinal end face and which houses the heating element 28.

The consumable 1/1'/1"'is insertable into the chamber 35 within the device housing 34 such that the heating element 28 is received in the housing 16 (e.g. within the planar recess 27 or cuboid recess 10 within the reconstituted tobacco) via the upstream longitudinal end face 19 of the housing 16. The downstream longitudinal end wall 21 of the housing 16 protrudes from the device housing 34.

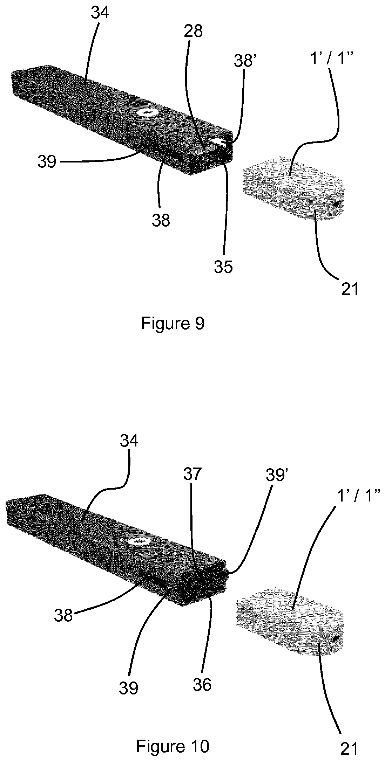

The device 30 further comprises a sealing plate 36 movable (slidable in a axial direction) from a first position (shown in figure 9) in which the aperture at the upstream longitudinal end face of the device housing 34 is open, to a second position (shown in Figure 10) in which the aperture is at least partially sealed by the sealing plate 36.

In the first position, the sealing plate 36 forms a base of the chamber 35 with the heating element 28 extending towards the aperture through the sealing plate 36. The sealing plate 36 has a slit 37, so that as it moves from the first to the second position, the heating element 28 passes through the slit.

The device housing 34 has two opposing channels 38, 38' and the sealing plate 36 comprises two opposing transverse tabs 39, 39' extending from the sealing plate 36 through the channels 38, 38' to rest on an exterior of the device housing 34. The transverse tabs 39, 39' may be used to manually move the sealing plate 36 between the first and second positions.

The consumable 1/1'/1" is inserted when the sealing plate 36 is in its first position. The heating element 28 lies within the cuboid recess 10 or the planar recess 27 and the user activates the heating element 28 e.g. by an actuator button located on the device housing 34. The device housing 34 may also comprise an indicator showing when the heating element 28 had reached the correct temperature (250°C).

The user then places the downstream longitudinal end wall 21 of the consumable 1/1'/1" into their mouth and draws on the consumable 1/1'/1" in order to inhale an aerosol containing nicotine.

After use, the sealing plate 36 is moved to its second position which forces the consumable 1/1'/1" from the chamber 35 and ultimately blocks the aperture at the upstream longitudinal end face of the device housing 34 so that the user is prevent from contacting the hot heating element 28.

The device 30 may further comprise a cap 40 e.g. a magnetic cap for sealing the aperture at the upstream longitudinal end face of the device housing e.g. when the device is not in use for an extended period. The cap 40 may have a recess on its underside such that the aperture can be sealed with a consumable 1/1'/1" in situ.

Figures 14 shows a perspective internal view of an eighth embodiment of a consumable 1"'.

The planar substrate 2'" comprises a planar upper surface 3' and a planar lower surface 53 spaced by opposing longitudinally-extending transverse surfaces 54a, 54b. The depth of the substrate 2'" (between the upper and lower surfaces, 3', 53) and the width of the substrate 2'" (between the opposing transverse surfaces 54a, 54b) are unequal with the width being greater than the depth.

The opposing transverse surfaces 54a, 54b each comprise a longitudinally-extending upper concave portion 55a, 55b and lower concave portion 56a, 56b which meet at a longitudinally-extending ridge 57a, 57b.

The concave portions are spaced from the upper surface 3' and lower surface 53 by opposing convex portions 58a, 58a', 58b, 58b' such that the transverse cross-section through the substrate 2'" is a modified obround where the opposing transverse surfaces 54a, 54b each take the form of a curly brace/bracket i.e. "{" and "}".

The length of the substrate 2'" (between an upstream end face 59 and a downstream end face 60) is around 12 mm long. The width of the substrate 2'" (between opposing transverse surfaces 54a, 54b) may be around 12 mm. The depth of the substrate 2'" (between the upper and lower surfaces) may be around 6 mm.

The substrate 2'" is formed of cast leaf slurry recon tobacco. It may alternatively be formed as extruded tobacco e.g. with added flavouring.

The consumable further comprises a planar filter 4'. The filter 4' comprises a substantially planar upper surface 61 and a substantially planar lower surface 62 equally spaced by opposing longitudinally-extending transverse surfaces 63a, 63b.

The opposing transverse surfaces 63a, 63b each comprise a substantially convex surface (a semi-circular surface) such that the planar filter 4' has a substantially obround transverse cross section i.e. the filter 4' is an obround cylindrical filter.

The filter 4' has greater width and length than depth. The length is around 22mm and the width is around 12 mm. The depth is around 6mm.

The filter 4' has a hollow bore 64. The hollow bore 64 extends from an upstream longitudinal end face 5' of the filter 4' to a downstream longitudinal end face 6' of the filter 4'.

The hollow bore 64 has an obround transverse cross sectional area. The bore 64 has a uniform transverse cross-sectional area. The bore is 64 dimensioned such that there is a thickness of filter material of around 1.5mm from the bore to the upper lower surfaces 61, 62 and the opposing transverse surfaces 63a, 63b.

The upstream longitudinal end face 5' of the filter 4' faces and abuts a downstream longitudinal end face 65 of the substrate 2"'.

The filter 4' is comprised of cellulose acetate or polypropylene tow. The filter 4' is circumscribed with a paper plug wrap (not shown).

The substrate 2'" and filter 4' are contained within a rigid bagasse housing 16'.

The housing 16' comprises upper and lower walls 22', 23' (see Figure 15) spaced by opposing longitudinally-extending transverse surfaces 24a', 24b'. The housing 16' has a wall thickness in the range of around 0.8 mm.

The upper and lower walls 22', 23' are substantially planar and equally spaced by the transverse surfaces 24a', 24b', (i.e. the upper and lower walls 22', 23' are parallel to one another).

The opposing transverse surfaces 24a', 24b' each comprise upper and lower concave portions 66a, 66a', 66b, 66b' which meet at a longitudinally-extending ridge 67.

The concave portions 66a, 66a', 66b, 66b' are spaced from the upper and lower surfaces by opposing convex portions 68a, 68a', 68b, 68b' such that the transverse cross-section of the housing 16' is a modified obround.

The chamber within and defined by the inner surfaces of the housing walls 22', 23', 24a', 24b' is a modified obround cylindrical chamber, i.e. the transverse cross-section of the chamber within the housing 16' matches the transverse cross section of the substrate 2"'.

The housing 16' may have a length of around 42 mm, a height of around 6 mm and a width of around 15mm.

The housing 16' has open upstream longitudinal end face which may be sealed by a metallic foil or a plastic membrane (not shown). This foil or membrane obscures the substrate 2'" from view.

The downstream longitudinal end of the housing 16' is shown in Figure 15. The downstream longitudinal end wall 21' conceals the filter 4' from view by the user.

Although the downstream longitudinal end wall 21' comprises a mouthpiece aperture 69, this is small enough (with a maximum depth of 0.6 mm and a width of 7.3 mm) that visual inspection of the filter 4" is significantly impeded.

The downstream longitudinal end wall 21', the upper wall 22', lower wall 23' and transverse side walls 24a', 24b' are coated with a hydrophobic coating or textured to provide a hydrophobic surface. In other embodiments, the downstream longitudinal end wall 21', the upper wall 22', lower wall 23' and transverse side walls 24a', 24b' comprise a layer of bagasse that has a lower porosity (e.g. around 5%) than the bagasse used to form the housing.

The consumable 1'" is heated in a heat not burn device. The device may comprise a heating element e.g. a planar heating element, for penetrating the substrate 2'" through the upstream longitudinal end face 59. For example, the device may be as described in relation to figures 8-10.

In other embodiments, the device may comprise one or more (e.g. two) external heating elements e.g. planar external heating elements for abutment against and heating of the substrate through the upper and lower walls 22', 23' of the housing 16'.

Figures 16a-16g shows various alternative transverse cross sections of the substrate, filter or housing. The substrate/filter transverse cross-sections could each comprise a hollow core recess which could have the same or different transverse cross section.

Figure 16a shows a substrate with planar upper and lower surfaces and convex (semi-circular) transverse surfaces such that the substrate has an obround transverse cross-section.

Figure 16b shows a substrate with planar upper and lower surfaces and concave (semi-circular) transverse surfaces.

Figure 16c shows a substrate which is similar to the substrate shown in Figure 15 except that there are no convex portions joining the upper and lower surfaces and the concave portions.

Figure 16d shows a substrate which has an oval transverse cross-sectional area.

Figure 16e shows a substrate with curved (convex) upper and lower surfaces and planar transverse surfaces such that the substrate has a truncated oval transverse cross-sectional area.

Figure 16f shows a substrate the same as Figure 16c except with curved (convex) upper and lower surfaces.

Figure 16g shows a substrate the same as Figure 16b except with curved (convex) upper and lower surfaces.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

## Claims

1. A heat not burn (HNB) consumable comprising an aerosol-forming substrate housed within a moulded or extruded housing, the housing having an inner surface facing the substrate and an opposing outer surface wherein at least a portion of at least one of the inner and outer surfaces of the housing comprises a moisture resistant surface.

2. A consumable according to claim 1 wherein the moisture resistance surface comprises a hydrophobic coating.

3. A consumable according to claim 2 wherein the hydrophobic coating is a food grade hydrophobic coating and/or biodegradable.

4. A consumable according to any one of the preceding claims wherein the moisture resistant surface comprises a textured hydrophobic surface.

5. A consumable according to any one of the preceding claims wherein the moisture resistant surface comprises a layer of material having a density greater than the density of the material used to form the housing.

6. A consumable according to claim 5 wherein the layer of material has a porosity of less than 20%.

7. A consumable according to claim 5 or 6 wherein the layer of material comprises the same material used to form the housing.

8. A consumable according to any one of the preceding claims wherein the hydrophobic coating/surface or denser layer of material is provided on the inner surface of the housing.

9. A consumable according to claim 8 wherein the inner surface of the housing is textured.

10. A consumable according to any one of the preceding claims wherein the hydrophobic coating/surface or denser layer of material is provided on the outer surface of the housing.

11. A consumable according to claim 10 wherein the housing comprises an outlet/mouthpiece aperture formed at a downstream lateral end of the housing and the hydrophobic coating/surface is provided on the outer surface of the housing surrounding the outlet/mouthpiece aperture.

12. A consumable according to any one of the preceding claims wherein the housing comprises upper and lower walls spaced by opposing longitudinally-extending transverse walls wherein the width of the housing is greater than the depth of the housing and wherein the housing has at least one curved or rounded wall.

13. A heat not burn (HNB) system comprising:
a heat not burn consumable according to any one of claims 1 to 12; and
a device comprising at least one heating element.

14. A system according to claim 13 wherein the device comprises a device housing having a cavity for receiving the consumable wherein the at least one heating element projects into or surrounds the cavity.

15. A method of using a heat not burn system according to claim 13 or 14, the method comprising:
inserting the consumable into the device; and
heating the consumable using the heating element.
